Europäisches Patentamt

European Patent Office

Office européen des brevets

(19)

(11) Publication number: **0 261 887**
**A2**

(12) **EUROPEAN PATENT APPLICATION**

(21) Application number: **87308282.0**

(22) Date of filing: **18.09.87**

(51) Int. Cl.⁴: **G01N 27/40** , G01N 33/50

(30) Priority: **22.09.86 GB 8622788**

(43) Date of publication of application:
**30.03.88 Bulletin 88/13**

(84) Designated Contracting States:
**CH DE FR LI**

(71) Applicant: **UNITED KINGDOM ATOMIC
ENERGY AUTHORITY**
**11 Charles II Street**
**London SW1Y 4QP(GB)**

(72) Inventor: **Williams, David Edward**
**41 Oxford Road**
**Abingdon Oxon(GB)**
Inventor: **Fleischmann, Martin**
**Bury Lodge Duck Street**
**Tisbury Wiltshire(GB)**

(74) Representative: **Bennett, Clive Stephen**
**United Kingdom Atomic Energy Authority**
**Patent Branch 11, Charles II Street**
**London, SW1Y 4Qp(GB)**

(54) **Sensor.**

(57) A sensor suitable for the detection of chemical species (e.g. biochemical species) includes a membrane having an ionophore such that gated ion transport may occur through the membrane. The membrane and/or the ionophore is/are such that a dynamic characteristic of conductance through the membrane may be modified when a species to be detected is present. Means are also provided for measuring a dynamic characteristic of conductance through the membrane.

The sensor may be used to detect species at very low concentrations and finds application in immunoassay.

EP 0 261 887 A2

## SENSOR

The present invention relates to sensors and more pariculary to sensors suitable for the detection of chemical species (e.g. biochemical species).

According to one aspect of the present invention there is provided a sensor which includes a membrane having an ionophore such that gated ion transport may occur through the membrane, said membrane and/or said ionophore being such that a dynamic characteristic of conductance through the membane may be modified when a species to be detected is present, and means for measuring a dynamic characteristic of conductance through the membrane.

A feature of gated ion transport detectable in particular at low concentrations of ionophore is randomly occurring current pulses representing corresponding fluctuation in conductance through the membrane. These fluctuations may be characterised in a number of ways, such as by frequency, spectral power density, variance in the current. Any such measurable characterstic of the fluctuations is referred to herein as a dynamic characteristic of conductance.

In accordance with one embodiment of the present invention there is provided a sensor which includes a membrane having an ionophore such that gated ion transport may occur through the membrane, said ionophore being capable of interacting with a species to be detected, when a species to be detected is present, such that dynamic characteristics of conductance through the membrane are modified, and means for measuring the dynamic characteristics of conductance through the membrane.

In said embodiment the ionophore may be, for example, such that it may be associated with the species to be detected (e.g. by being chemically or physically linked thereto) so as to modify dynamic characteristics of conductance through the membrane. For example, the ionophore may be such that it is an antigen specific to a species to be detected comprising an antibody. Thus, the binding of antibody to the ionophore, which comprises the antigen, may give rise to modifications in dynamic characteristics of conductance through the membrane thereby allowing the specific binding event of antibody to antigen to be detected by monitoring a dynamic characteristic of conductance through the membrane.

In another embodiment of the present invention there is provided a sensor which includes a membrane having an ionophore such that gated ion transport may occur through the membrane, a modifying species capable of modifying a dynamic characteristic of conductance through the membrane when a species to be detected is present and means for measuring a dynamic characteristic of conductance through the membrane.

The modifying species may be, for example, associated with the ionophore (e.g. by being chemically or physically linked thereto). Thus, for example, an antigen may be associated with an ionophore to given an antigen-labelled ionophore and the labelled ionophore may be used to modify a dynamic characteristic of conductance through the membrane when its corresponding antibody comprises the species to be detected.

By way of further example the modifying species may be associated with parts or constituents of the membrane other than the ionophore. Thus, for example, the membrane may contain an antibody as the modifying species; the binding of an antigen to the antibody may then be used to modify a dynamic characteristic of conductance through the membrane. Conversely the membrane may contain an antigen as the modifying species which, upon binding with an antibody which comprises the species to be detected, may modify a dynamic characteristic of conductance through the membrane.

Where the membrane is, for example, a lipid layer, modifying species comprising an antigen may be introduced thereto to produce antigen-labelled lipids which upon interaction with the corresponding antibody will give rise to modification of a dynamic characteristic of conductance. It will be appreciated that the modifying species may, for example, be associated with the membrane by being chemically or physically linked thereto.

The membrane may be, for example, a bi-layer lipid membrane dividing two solutions or a bi-layer membrane supported on a porous substrate such as a hydro-gel or a millipore filter.

Lipid membranes may be formed by any suitable method. By way of example, membranes have been formed in accordance with the present invention from a mixture of cholesterol and glycerol mono-oleate. N-hexadecane has been used as a solvent for cholesterol and glycerol mono-oleate in the formation of lipid membranes in accordance with the present invention.

By way of example, in accordance with the present invention, the protein haemocyanin has been used as an ionophore in a bi-layer lipid membrane. By way of further example haemocyanin has been labelled with biotin which is capable of specific binding with strepavidin and a membrane containing haemocyanin labelled with biotin used to detect strepavidin in solution by monitoring a dynamic characteristic of conductance through the membrane in respectively the absence and the presence of strepavidin.

Also in accordance with the present invention, by way of example, gramicidin has been labelled with biotin and used in a bi-layer lipid membrane to detect strepavidin by means of monitoring a dynamic characteristic of conductance through the membrane in, respectively, the absence and the presence of strepavidin. It will be appreciated that in the presnce of strepavidin a specific interaction occurs between strepavidin and biotin thereby modifying the dynamic characteristics of conductance.

Dynamic characteristics of conductance through the membrane may be measured by any suitable technique. Thus, for example, spectral power density, event frequency, auto-correlation function, variance of signal, probability density function of current, average time for which the conductance assumes a specified value (static lifetime), frequency with which the conductance crosses some specified level, or correlation function of level-crossing times, may be used in measuring dynamic characteristics of conductance through the membrane.

It will be appreciated that in accordance with the present invention the concentration of ionophore is sufficiently low to enable gated ion transport to be observed.

Examples of ionophores which may be used in accordance with the present invention are gramicidin, haemocyanin, alamethicin, mellitin and amphotericin B.

According to another aspect of the present invention there is provided a method for the detection of a species which method comprises contacting a species to be detected with a sensor including a membrane having an ionophore such that gated ion transport may occur through the membrane, said membrane and/or said ionophore being such that a dynamic characteristic of conductance through the membrane may be modified in the presence of the species to be detected, and measuring a dynamic characteristic of conductance through the membrane.

By way of example in accordance with the present invention aqueous solutions of an inorganic salt may be provided on each side of a membrane having an ionophore in accordance with the present invention and the presence of a species to be detected may be sensed by detecting the effect on passage of ions from one solution through the membrane to the other solution, said . detecting being by means of measuring a dynamic characteristic of conductance through the membrane.

It will be appreciated that the solutions may also contain ionophores in solution. An electrode can be positioned in each of the solutions and may be connected, for example, to a device for measuring a dynamic characteristic of conductance through the membrane.

By way of example a suitable solution for providing on each side of the membrane is an aqueous potassium chloride solution.

It will be appreciated that in accordance with the present invention a very low concentration of species to be detected may be sensed (e.g. at concentrations of approximately $10^{-12}$ moles per litre). In general the ionophore concentration in a solution in contact with the membrane may be of the order of a few nanograms per millilitre.

Immunoassay measurements may be carried out using a sensor in accordance with the present invention.

The invention will now be further described, by way of example only, as follows:

Example

A PTFE separator was provided between two chambers machined from perspex and a small hole (0.5 mm diameter) was provided in the separator to connect the two chambers. The chambers were filled with potassium chloride solution (0.1M) and a chlorided silver foil was placed in each chamber to act as electrodes (the silver foils had been previously provided with a porous coating of silver chloride by prior electrolytic treatment in a solution of potassium chloride). A lipid membrane precursor solution, comprising 4 wt % cholesterol and 4 wt % glycerol mono-oleate in n-hexadecane, was brushed over the hole in order to form a bi-layer lipid membrane separating the two chambers.

Solutions of keyhole limpet haemocyanin (Sigma Chemical Company) and of haemocyanin labelled with biotin (Sigma Chemical Company) having concentrations of approximately 1μ g/ml, were prepared by dissolving 1 mg of solid haemocyanin in water (8 ml) then diluting further. 1μ l of each solution was added to one chamber (volume 15 ml) to give a solution containing a total of $2 \times 10^{-11}$g of haemocyanin (ionophore) per millilitre. Application of a bias of -40 mV (with the side to which ionophore added negative) then caused gated ion transport to begin as ionophore became incorporated into the membrane.

Dynamic characteristics of the conductance were monitored by measurement of the current and recording of the autocorrelation function spectral power density, variance of the current and of the average frequency of the randomly occurring current pulses which are characteristic of the gated transport. The addition of 1μ l of an aqueous solution of strepavidin to the same chamber to which the ionophore had been added (8 x 10⁻⁸ g/ml; a total addition of 8 x 10⁻¹¹g giving a concentration in the chamber of 5 x 10⁻¹² g/ml) gave a very marked reduction in the pulse frequency, (which dropped from approximately 2 events per second to about 0.1 - 0.2 events per second), in the autocorrelation function and in the variance of the current, corresponding to the interaction of strepavidin with the biotin-labelled ionophore.

**Claims**

1. A sensor which includes a membrane having an ionophore such that gated ion transport may occur through the membrane, said membrane and/or said ionophore being such that a dynamic characteristic of conductance through the membrane may be modified when a species to be detected is present, and means for measuring a dynamic characteristic of conductance through the membrane.

2. A sensor according to claim 1 wherein said ionophore is capable of interacting with a species to be detected, when present, such that dynamic characteristics of conductance through the membrane are modified.

3. A sensor according to claim 2 wherein the ionophore is capable of being chemically or physically linked to the species to be detected.

4. A sensor according to claim 3 wherein the ionophore is an antigen specific to a species to be detected comprising an antibody.

5. A sensor according to claim 1 further including a modifying species capable of modifying a dynamic characteristic of conductance through the membrane when a species to be detected is present.

6. A sensor according to claim 5 wherein the modifying species is associated with the ionophore.

7. A sensor according to claim 6 wherein the modifying species is an antigen and the species to be detected comprises its corresponding antibody.

8. A sensor according to claim 5 wherein the modifying species is associated with parts or constituents of the membrane other than the ionophore.

9. A sensor according to claim 8 wherein the membrane contains an antibody as the modifying species whereby the binding of an antigen thereto is capable of modifying a dynamic characteristic of conductance through the membrane.

10. A sensor according to claim 8 wherein the membrane contains an antigen as the modifying species whereby the binding of an antibody thereto is capable of modifying a dynamic characteristic of conductance through the membrane.

11. A sensor according to claim 10 wherein the membrane is a lipid layer containing a modifying species comprising an antigen to produce an antigen-labelled lipid.

12. A sensor according to claim 1 wherein the ionophore is haemocyanin or gramicidin and the membrane is a bi-layer lipid membrane.

13. A sensor according to claim 12 for detecting strepavidin wherein the haemocyanin or gramicidin is labelled with biotin.

14. A sensor according to any of the preceding claims wherein the means for measuring a dynamic characteristic of conductance through the membrane is capable of measuring one or more of spectral power density, event frequency, auto-correlation function, variance of signal, probability density function of current, static lifetime, frequency with which the conductance has a specified value and correlation function of level-crossing times.

15. A method for the detection of a species which method comprises contacting a species to be detected with a sensor including a membrane having an ionophore such that gated ion transport may occur through the membrane, said membrane and/or said ionophore being such that a dynamic characteristic of conductance through the membrane may be modified in the presence of the species to be detected, and measuring a dynamic characteristic of conductance through the membrane.

16. A method according to claim 15 wherein aqueous solutions of an inorganic salt are provided on each side of the membrane and the presence of a species to be detected is sensed by detecting the effect on passage of ions from one solution through the membrane to the other solution, said detecting being by means of measuring a dynamic characteristic of conductance through the membrane.

17. A method according to claim 16 wherein one or both of the solutions contain ionophores dissolved therein.

18. A method according to claim 16 or 17 wherein an electrode is positioned in each solution and is connected to a device for measuring a dynamic characteristic of conductance through the membrane.

19. A method according to any of claims 16 to 18 wherein each solution is an aqueous potassium chloride solution.

20. A method according to claim 17 and to claims 18 and 19 when dependent on claim 17 wherein the ionophore concentration in one or both of the solutions is of the order of a few nanograms per millilitre.